# EUROPEAN PATENT APPLICATION

(11) **EP 4 261 202 A1**
(43) Date of publication of application: **18.10.2023**
(21) Application number: 21902559.0
(22) Date of filing: 06.12.2021
(51) Int. Cl.: C07C 229/64, C07C 227/42

(54) **2-HYDROXY-5-[2-(4-(TRIFLUOROMETHYLPHENYL)ETHYLAMINO)]BENZOIC ACID CRYSTAL FORMS AND PREPARATION METHOD THEREFOR**

(30) Priority: 08.12.2020 CN 202011443509
(71) Applicant: GNT Pharma Co., Ltd, Yongin-si, Gyeonggi-do, 17096 (KR)
(72) Inventor: ZHANG, Guoqing, Hangzhou, Zhejiang 310018 (CN); ZHUANG, Chenghan, Dongyang, Zhejiang 322118 (CN); WANG, Lei, Dongyang, Zhejiang 322118 (CN); GWAG, Byoung Joo, Yongin-si, Gyeonggi-do 17096 (KR); AHN, Chun San, Yongin-si, Gyeonggi-do 17096 (KR); JIN, Jing Yu, Yongin-si, Gyeonggi-do 17096 (KR); XU, Xinliang, Dongyang, Zhejiang 322118 (CN)
(74) Representative: Cabinet Nony
(86) International application number: PCT/CN2021/135807
(87) International publication number: WO 2022/121854

(57) **Abstract**

The present invention relates to the technical field of pharmaceutical crystal forms, and specifically relates to 2-hydroxy-5-[2-(4-(trifluoromethylphenyl)ethylamino)]benzoic acid crystal forms and a preparation method therefor. Crystal form I is a monoclinic crystal system, which has a Pc space group and can be obtained by means of crystallization methods of slow cooling, evaporating the solvent at a constant temperature, evaporating the solvent at an increased temperature, or adding an anti-solvent. Crystal form II is a triclinic crystal system, which has a P1 space group and can be obtained by crystallization methods such as rapid cooling or freeze-drying. The advantages of the two crystal forms and the preparation method therefor of the present invention are as follows: 1) the process is simple, costs are low, and the yield exceeds 90%; and 2) the crystal forms of the crystal forms I and II have high purity, the crystal shapes thereof are intact, and have excellent fluidity, facilitating preparation, particularly the preparation of a pharmaceutical preparation for preventing and/or treating degenerative diseases of the central nervous system; in addition, the two crystal forms have a better apparent solubility than that of raw materials. (Figure 1)

## Description

### CROSS-REFERENCE TO RELATED APPLICATIONS

This application claims the priority of a patent application for invention with an Application No. 202011443509.5, filed with the China National Intellectual Property Administration on December 8, 2020, and titled with "2-HYDROXY-5-[2-(4-(TRIFLUOROMETHYLPHENYL)ETHYLAMINO)]BENZOIC ACID CRYSTAL FORMS AND PREPARATION METHOD THEREFOR", which is herein incorporated by reference in its entirety.

### FIELD OF THE INVENTION

The present disclosure relates to the field of pharmaceutical crystalline forms, specifically to crystalline forms I and II of 2-hydroxy-5-[2-(4-(trifluoromethylphenyl) ethylamino)]benzoic acid, a preparation method therefor and use thereof.

### BACKGROUND OF THE INVENTION

Senile Dementia, also known as Alzheimer's disease (AD), is a degenerative disease of the central nervous system mainly characterized by progressive cognitive impairment and memory impairment, and dementia is its most prominent mental symptom. Since the etiology and pathogenesis of the disease are still unclear, there is still a lack of specific etiological treatment methods. However, according to years of research by medical scientists, there are many medicines that have a good effect on improving the memory ability and cognitive function of senile dementia patients and delaying aging. The research and development of medicines for the treatment of senile dementia has attracted great attention from the medical circles all over the world. With the continuous deepening of research on the neurophysiology, biochemistry, and pharmacology of the elderly, the development and research of related medicines have also made continuous progress.

GNT Pharma Co., Ltd. of Republic of Korea has developed a compound that has a good therapeutic effect on the treatment of neurological diseases such as Alzheimer's disease and Parkinson's disease, which has a Chinese name of 2- 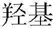 -5-[2-(4-( 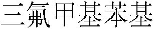 ) 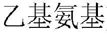 )] 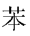 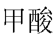 , an English name of 2-hydroxy-5-[2-(4-(trifluoromethylphenyl)ethylamino)]benzoic acid, and a structure shown below:

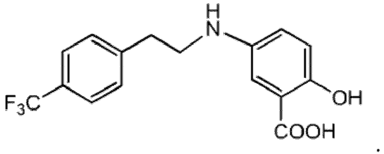

A Chinese patent application for invention CN 101874016 A of GNT Pharma Co., Ltd. discloses the compound and its preparation technology. Clinical trials have shown that the compound has a good inhibitory effect on Alzheimer's disease, but its appearance and micromorphology have not been described.

### SUMMARY OF THE INVENTION

### TECHNICAL PROBLEM

The present disclosure provides crystalline forms I and II of 2-hydroxy-5-[2-(4-(trifluoromethylphenyl)ethylamino)]benzoic acid, which are suitable for industrial production, have large particle size, intact structure and good fluidity for the prepared crystal particles, and facilitate preparation, and provides a preparation method therefor and medical use thereof.

### TECHNICAL SOLUTION

A series of 2-hydroxy-5-[2-(4-(trifluoromethylphenyl)ethylamino)]benzoic acid products were prepared through different technological processes in the present disclosure, and it is found that their solids show two relatively stable crystalline forms, named crystalline forms I and II.

In a first aspect, the present disclosure provides crystalline form I of 2-hydroxy-5-[2-(4-(trifluoromethylphenyl)ethylamino)]benzoic acid, and its crystallographic parameters are as follows:
crystal system: monoclinic crystal system;
space group: Pc;
cell parameters: a=3.83880(10)Å, b=8.82524(12)Å, c=11.62736(9)Å, α=90°, β=98.7472°, γ=90°; and
number of molecules per unit cell: Z=2.

In addition, the X-ray powder diffraction spectrum (XRPD) of the crystalline form I shows characteristic peaks at 2θ values of 16.1±0.2°, 19.4±0.2°, 22.9±0.2°, 27.3±0.2° and 28.4±0.2°.

Further, the XRPD of the crystalline form I shows characteristic peaks at 2θ values of at least one of 6.9±0.2°, 13.6±0.2°, 20.5±0.2°, 21.9±0.2°, 22.6±0.2°, 25.1±0.2°, 25.7±0.2° and 34.3±0.2°.

Still further, the XRPD of the crystalline form I shows characteristic peaks at 2θ values of at least one of 12.9±0.2°, 17.4±0.2°, 29.0±0.2°, 29.7±0.2°, 31.4±0.2°, 34.8±0.2°, 36.0±0.2°, 36.5±0.2° and 39.1±0.2°.

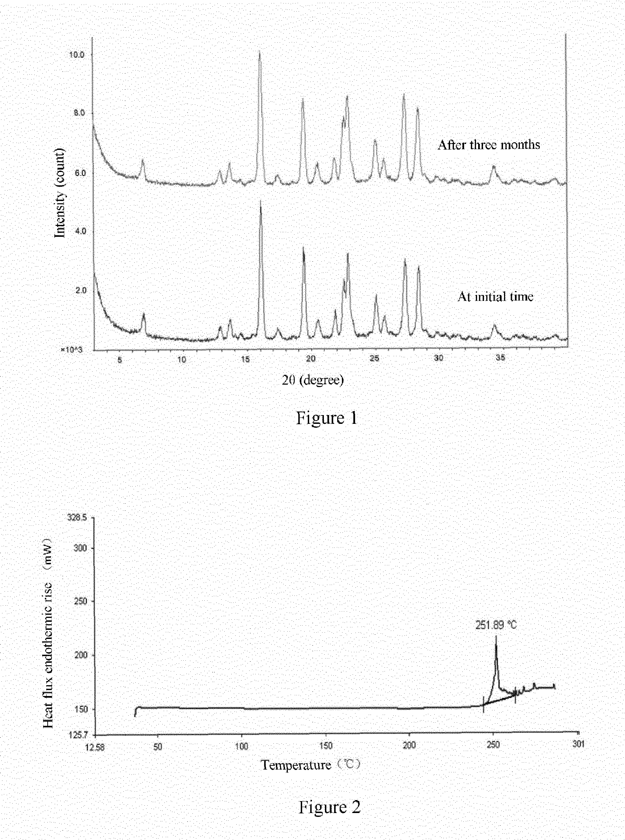

Still further, the XRPD of the crystalline form I is shown in FIG. 1.

In addition, the differential scanning calorimetry analysis graph (DSC) of the crystalline form I shows an endothermic peak at 249~252°C.

Further, the DSC of the crystalline form I is shown in FIG.2.

In addition, the morphology of the crystalline form I is irregular block with an average particle size of 50 µm.

In a second aspect, the present disclosure provides crystalline form II of 2-hydroxy-5-[2-(4-(trifluoromethylphenyl)ethylamino)]benzoic acid, and its crystallographic parameters are as follows:
crystal system: triclinic crystal system;
space group: P1;
cell parameters: a=4.47850(8)Å, b=6.31509(15)Å, c=13.34065(22)Å, α=89.9804(7)°, β=79.7108(12)°, γ=80.6128(8)°; and
number of molecules per unit cell: Z=2.

In addition, the X-ray powder diffraction spectrum (XRPD) of the crystalline form II shows characteristic peaks at 2θ values of 6.8±0.2°, 13.5±0.2°, 27.2±0.2° and 28.3±0.2°.

Further, the XRPD of the crystalline form II shows characteristic peaks at 2θ values of at least one of 16.0±0.2°, 20.4±0.2°, 22.4±0.2° and 34.2±0.2°.

Still further, the XRPD of the crystalline form II shows characteristic peaks at 2θ values of at least one of 19.3±0.2°, 22.7±0.2°, 23.1±0.2°, 24.5±0.2°, 25.0±0.2° and 26.0±0.2°.

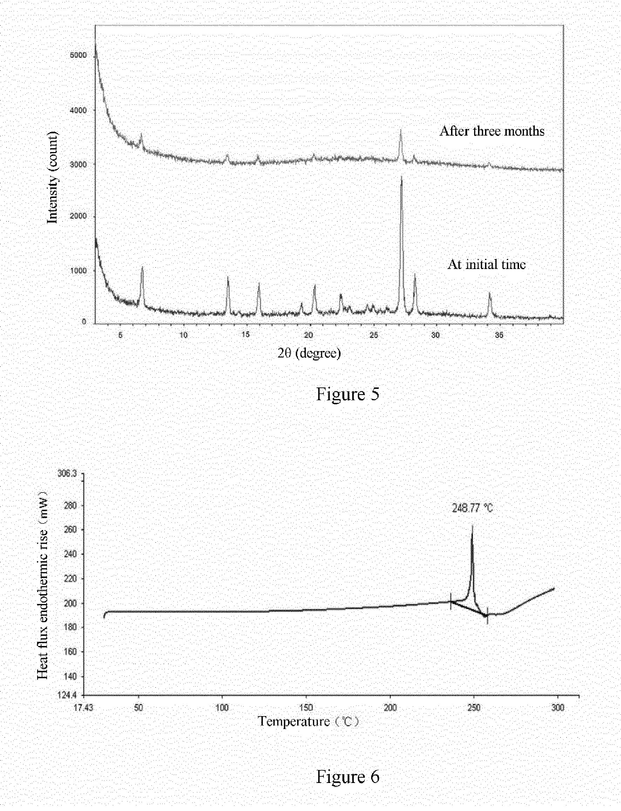

Still further, the XRPD of the crystalline form II is shown in FIG. 5.

In addition, the differential scanning calorimetry analysis graph (DSC) of the crystalline form II shows an endothermic peak at 247~250°C.

Further, the DSC of the crystalline form II is shown in FIG.6.

In addition, the morphology of the crystalline form II is a regular rod with an average particle size of 18 µm.

In a third aspect, the present disclosure provides a method for preparing the above crystalline form I, which comprises:
adding a crude product of 2-hydroxy-5-[2-(4-(trifluoromethylphenyl)ethylamino)]benzoic acid to a good solvent, stirring and heating until it is completely dissolved, filtering while it is hot, and subjecting the filtrate to slow cooling crystallization, evaporation of the solvent at a constant temperature for crystallization, evaporation of the solvent at an increased temperature for crystallization or addition of an anti-solvent for crystallization to obtain the crystalline form I.

Further, the good solvent is at least one selected from the group consisting of methanol, ethanol, isopropanol, tetrahydrofuran and dimethyl sulfoxide, and preferably at least one selected from the group consisting of ethanol and isopropanol.

Further, the usage ratio of the crude product to the good solvent is 1 g: 5~300 mL.

Further, the target temperature of the heating is 50~80°C.

Further, the target temperature of the slow cooling crystallization is 0°C or lower, and the cooling rate is 3°C/min or less.

Further, the ambient temperature of evaporating the solvent at a constant temperature for crystallization is 10~30°C.

Further, the target temperature of evaporating the solvent at an increased temperature for crystallization is 80°C or higher.

Further, the anti-solvent used for addition of an anti-solvent for crystallization is at least one selected from the group consisting of water, acetonitrile, ethyl ether, acetone, petroleum ether, n-hexane, and n-heptane, preferably water and petroleum ether.

Still further, the volume ratio of the anti-solvent to the good solvent is 1∼5:1, preferably 2:1.

In a fourth aspect, the present disclosure provides a method for preparing the above crystalline form II, which comprises:
adding a crude product of 2-hydroxy-5-[2-(4-(trifluoromethylphenyl)ethylamino)]benzoic acid to a good solvent, stirring and heating until it is completely dissolved, filtering while it is hot, and subjecting the resulting filtrate to rapid cooling crystallization or freeze-drying crystallization to obtain the crystalline form II.

Further, the good solvent is at least one selected from the group consisting of methanol, ethanol and tetrahydrofuran, preferably at least one selected from the group consisting of methanol and ethanol.

Further, the usage ratio of the crude product to the good solvent is 1 g: 5~300 mL.

Further, the target temperature of the heating is 50~80°C.

Further, the target temperature of the rapid cooling crystallization is 0°C or lower, and the cooling rate is 5/min or greater.

In a fifth aspect, the present disclosure provides a pharmaceutical composition comprising the above crystalline form I and/or crystalline form II, and at least one pharmaceutically acceptable carrier.

In a sixth aspect, the present disclosure provides the use of the above crystalline form I and/or crystalline form II in the preparation of a medicament for the prevention and/or treatment of degenerative diseases of the central nervous system.

Further, the degenerative disease of the central nervous system is at least one selected from the group consisting of Alzheimer's disease, Parkinson's disease, Huntington's disease and amyotrophic lateral sclerosis, preferably Alzheimer's disease.

### TECHNICAL EFFECTS

The advantages of the two crystalline forms (crystalline forms I and II) of 2-hydroxy-5-[2-(4-(trifluoromethylphenyl)ethylamino)]benzoic acid and the preparation method therefor provided in the present disclosure are as follows: 1) the process is simple, the cost is low, and the yield is as high as 90% or more; and 2) the crystalline forms of the crystalline forms I and II have high purity, intact crystal shape and good fluidity, thereby facilitating preparation, and have a better apparent solubility than that of raw materials.

### DESCRIPTION OF DRAWINGS

FIG. 1 is an XRPD spectrum of the crystalline form I of 2-hydroxy-5-[2-(4-(trifluoromethylphenyl)ethylamino)]benzoic acid prepared in Example 1, including the sample spectra at initial time and after three months of stability test.
FIG. 2 is a DSC graph of the crystalline form I of 2-hydroxy-5-[2-(4-(trifluoromethylphenyl)ethylamino)]benzoic acid prepared in Example 1.
FIG. 3 is a SEM photograph of the crystalline form I of 2-hydroxy-5-[2-(4-(trifluoromethylphenyl)ethylamino)]benzoic acid prepared in Example 1.
FIG. 4 is a particle size distribution diagram of the crystalline form I of 2-hydroxy-5-[2-(4-(trifluoromethylphenyl)ethylamino)]benzoic acid prepared in Example 1.
FIG. 5 is an XRPD spectrum of the crystalline form II of 2-hydroxy-5-[2-(4-(trifluoromethylphenyl)ethylamino)]benzoic acid prepared in Example 5, including the sample spectra at initial time and after three months of stability test.
FIG. 6 is a DSC graph of the crystalline form II of 2-hydroxy-5-[2-(4-(trifluoromethylphenyl)ethylamino)]benzoic acid prepared in Example 5.
FIG. 7 is a SEM photograph of the crystalline form II of 2-hydroxy-5-[2-(4-(trifluoromethylphenyl)ethylamino)]benzoic acid prepared in Example 5.
FIG. 8 is a particle size distribution diagram of the crystalline form II of 2-hydroxy-5-[2-(4-(trifluoromethylphenyl)ethylamino)]benzoic acid prepared in Example 5.
FIG. 9 is an XRPD superposition spectrum of the crystalline form I of 2-hydroxy-5-[2-(4-(trifluoromethylphenyl)ethylamino)]benzoic acid prepared in Examples 1 to 4.
FIG. 10 is an XRPD superposition spectrum of the crystalline form II of 2-hydroxy-5-[2-(4-(trifluoromethylphenyl)ethylamino)]benzoic acid prepared in Examples 5 to 6.

### DETAILED EMBODIMENTS

Firstly, the present disclosure provides two crystalline forms, i.e. crystalline form I and crystalline form II, of 2-hydroxy-5-[2-(4-(trifluoromethylphenyl)ethylamino)]benzoic acid.

Secondly, the present disclosure provides a method for preparing the above two crystalline forms.

Thirdly, the present disclosure provides a pharmaceutical composition comprising at least one of the above two crystalline forms.

Finally, the present disclosure provides the pharmaceutical use of at least one of the above two crystalline forms.

### Crystalline forms I and II of 2-hydroxy-5-[2-(4-(trifluoromethylphenyl)ethylamino)] benzoic acid

The crystalline forms I and II of the present disclosure can be characterized by methods such as X-ray powder diffraction (XRPD), differential scanning calorimetry analysis (DSC), and scanning electron microscopy (SEM), and parameter results such as crystal system, space group, cell parameters and number of molecules per unit cell can be obtained through powder polycrystalline indexing. The above method can adopt parameter settings in the conventional operation in the art, and can be adjusted or changed as appropriate according to the specific physical and chemical properties of a substance to be tested.

### X-ray powder diffraction

In the present disclosure, the experimental conditions of X-ray powder diffraction are as follows. The test was performed by an X-ray powder diffractometer (radiation source Cu-kα, λ = 1.5418Å), and the XRPD spectrum with 2θ value of 3 ~ 40° was scanned. The XRPD spectra of crystalline form I in Example 1 and crystalline form II in Example 2 are shown in FIG. 1 and FIG. 5, respectively, and the XRPD data are shown in Table 1. In order to test the stability of the crystalline form, the crystalline form I in Example 1 and the crystalline form II in Example 2 were subjected to powder diffraction test after being left under 40°C and 60% relative humidity (RH) conditions for three months, and the results are shown in FIG. 1 and FIG. 5, respectively.

**Table 1. XRPD data of crystalline forms I and II of the present disclosure**

| No. | Crystalline form I | | | Crystalline form II | | |
|---|---|---|---|---|---|---|
| | 2θ(°) | d value | Peak intensity (%) | 2θ(°) | d value | Peak intensity (%) |
| 1 | 6.916 | 12.7709 | 15.7 | 6.777 | 13.0321 | 28.8 |
| 2 | 12.880 | 6.8676 | 8.9 | 13.520 | 6.5440 | 27.2 |
| 3 | 13.641 | 6.4859 | 13.7 | 15.962 | 5.5478 | 23.5 |
| 4 | 16.081 | 5.5070 | 100.0 | 19.339 | 4.5860 | 8.3 |
| 5 | 17.359 | 5.1042 | 8.0 | 20.356 | 4.3590 | 21.8 |
| 6 | 19.440 | 4.5624 | 65.4 | 22.421 | 3.9621 | 13.8 |
| 7 | 20.538 | 4.3208 | 12.6 | 22.719 | 3.9107 | 5.0 |
| 8 | 21.877 | 4.0593 | 18.0 | 23.078 | 3.8507 | 5.4 |
| 9 | 22.560 | 3.9379 | 41.1 | 24.469 | 3.6349 | 5.9 |
| 10 | 22.899 | 3.8804 | 59.3 | 25.039 | 3.5534 | 5.1 |
| 11 | 25.060 | 3.5505 | 29.4 | 26.040 | 3.4191 | 5.0 |
| 12 | 25.718 | 3.4611 | 12.1 | 27.201 | 3.2757 | 100.0 |
| 13 | 27.340 | 3.2593 | 55.3 | 28.280 | 3.1531 | 28.9 |
| 14 | 28.420 | 3.1379 | 50.3 | 34.161 | 2.6225 | 17.7 |
| 15 | 28.959 | 3.0807 | 4.5 | | | |
| 16 | 29.744 | 3.0012 | 3.6 | | | |
| 17 | 31.441 | 2.8429 | 3.2 | | | |
| 18 | 34.281 | 2.6137 | 10.7 | | | |
| 19 | 34.778 | 2.5774 | 4.1 | | | |
| 20 | 36.039 | 2.4900 | 3.1 | | | |
| 21 | 36.503 | 2.4595 | 3.3 | | | |
| 22 | 39.120 | 2.3008 | 3.8 | | | |

### Differential scanning calorimetry analysis

In the present disclosure, the experimental conditions for differential scanning calorimetry analysis are as follows. 3~10 mg samples were accurately weighed and packaged in an aluminum crucible, and heated from 30°C to 300°C at a heating rate of 10°C/ min under nitrogen protection, and the related graphs were recorded. The DSC graphs of crystalline form I in Example 1 and crystalline form II in Example 2 are shown in FIG. 2 and FIG. 6, respectively, and both have obvious heating and melting processes. The crystalline form I has a melting point of 251.89°C, and the crystalline form II has a melting point of 248.77°C.

### Scanning electron microscopy

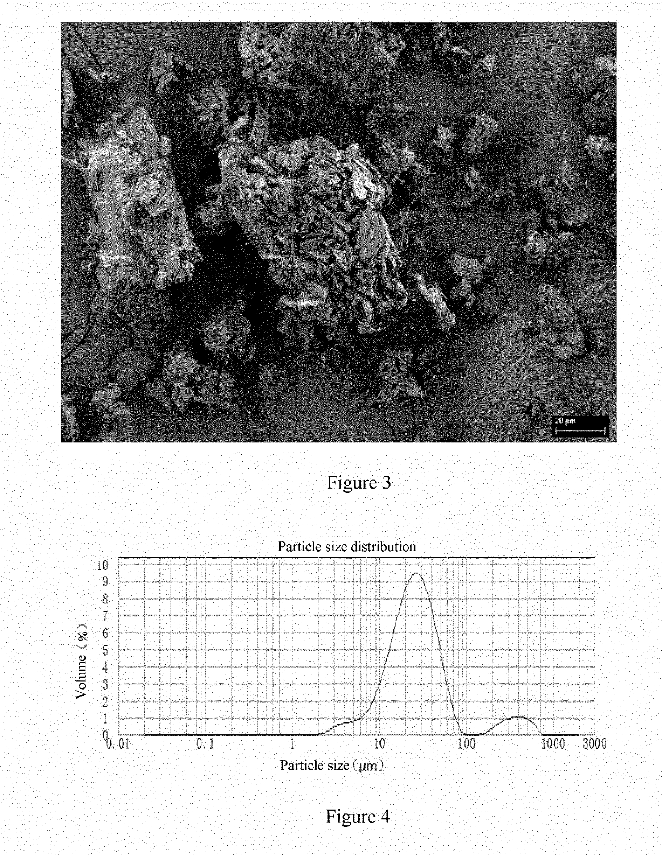

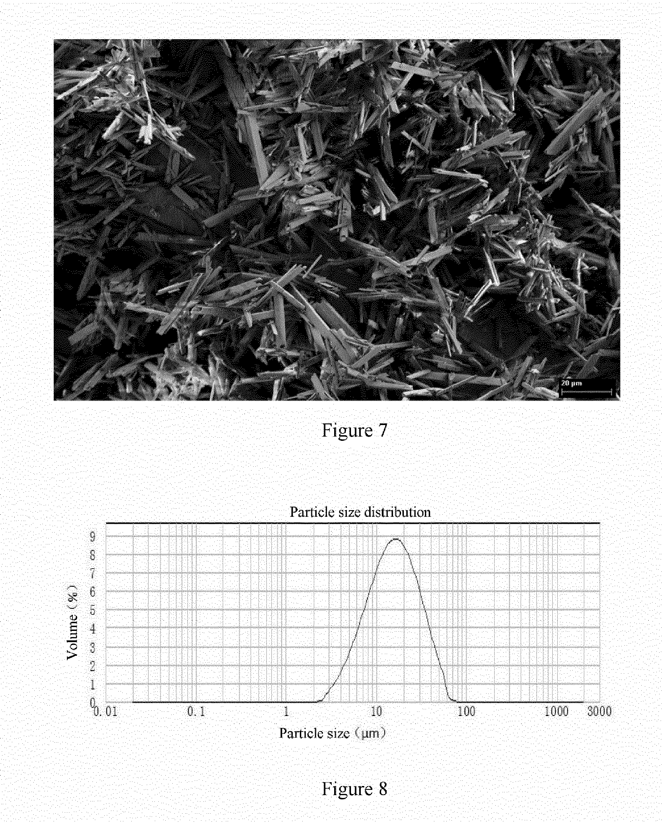

The SEM photographs of crystalline form I in Example 1 and crystalline form II in Example 2 are shown in FIG. 3 and FIG. 7, respectively. The morphology of the crystalline form I of the present disclosure is roughly irregular block with an average particle size of about 50 µm, as shown in FIG. 4. The morphology of the crystalline form II of the present disclosure is roughly regular rod with an average particle size of about 18 µm, as shown in FIG. 8.

### Preparation methods of crystalline forms I and II of 2-hydroxy-5-[2-(4-(trifluoromethylphenyl)ethylamino)]benzoic acid

The method for preparing crystalline forms I and II of 2-hydroxy-5-[2-(4-(trifluoromethylphenyl)ethylamino)]benzoic acid of the present disclosure obtains corresponding crystalline forms I and II products, by selecting the good solvent and recrystallization condition, finally determining a mixture of one or more selected from the group consisting of alcohols (such as methanol, ethanol, isopropanol, etc.), tetrahydrofuran and dimethyl sulfoxide as the good solvent, and through different crystallization treatment methods.

The crystalline form I of the present disclosure can be prepared by the following preparation method. A crude product of 2-hydroxy-5-[2-(4-(trifluoromethylphenyl)ethylamino)] benzoic acid was added to a good solvent (or a main solvent), stirred and heated until it is completely dissolved, and filtered while it was hot. The filtrate was subjected to slow cooling crystallization, evaporation of the solvent at a constant temperature for crystallization, evaporation of the solvent at an increased temperature for crystallization or addition of an anti-solvent for crystallization, thus obtaining the crystalline form I.

In one embodiment of the present disclosure, the good solvent for dissolving the crude product of 2-hydroxy-5-[2-(4-(trifluoromethylphenyl)ethylamino)]benzoic acid may be a single solvent or a mixture of two or more solvents in any ratio, and for example, may be at least one selected from the group consisting of methanol, ethanol, isopropanol, tetrahydrofuran and dimethyl sulfoxide. In a preferred embodiment of the present disclosure, the good solvent for dissolving the crude product of 2-hydroxy-5-[2-(4-(trifluoromethylphenyl)ethylamino)]benzoic acid may be at least one selected from the group consisting of ethanol and isopropanol.

As for the crystalline form I of the present disclosure, the target product can be obtained by various crystallization methods, such as slow cooling crystallization, evaporating the solvent at a constant temperature for crystallization, evaporating the solvent at an increased temperature for crystallization or adding an anti-solvent for crystallization, etc.

In one embodiment of the present disclosure, the crystalline form I can be prepared by slow cooling crystallization method. Specifically, the target temperature of the slow cooling crystallization may be 0°C or lower, that is, the dissolved and clear filtrate of the crude product was slowly cooled to a temperature of 0°C or lower, so as to form a supersaturated solution at a lower temperature, thereby achieving crystallization. In addition, the cooling rate of the slow cooling crystallization may be 3°C/min or less, that is, the temperature of the dissolved and clear filtrate of the crude product was decreased to the target temperature at a rate of decreasing 3°C per minute or slower.

In another embodiment of the present disclosure, the crystalline form I can be prepared by a method of evaporating the solvent at a constant temperature for crystallization. Specifically, the ambient temperature of evaporating the solvent at a constant temperature for crystallization may be 10~30°C, that is, the temperature of the dissolved and clear filtrate of the crude product was firstly decreased to the ambient temperature of 10-30°C, and then a supersaturated solution was formed by evaporating the solvent under the condition of constant ambient temperature, thereby achieving crystallization.

In another embodiment of the present disclosure, the crystalline form I can be prepared by a method of evaporating the solvent at an increased temperature for crystallization. Specifically, the target temperature of evaporating the solvent at an increased temperature for crystallization may be 80°C or higher, that is, the dissolved and clear filtrate of the crude product was heated to a temperature of 80°C or higher, and a supersaturated solution was formed by evaporating the solvent, thereby realizing crystallization. In a preferred embodiment of the present disclosure, evaporating the solvent at an increased temperature for crystallization can be carried out under a certain vacuum degree, so as to accelerate the solvent removal rate.

In another embodiment of the present disclosure, the crystalline form I can be prepared by a method of adding an anti-solvent for crystallization. Specifically, the anti-solvent (or secondary solvent) may be a single solvent or a mixture of two or more solvents in any ratio, and for example, may be at least one selected from the group consisting of water, acetonitrile, ethyl ether, acetone, petroleum ether, n-hexane and n-heptane. In a preferred embodiment of the present disclosure, the amount of the anti-solvent can be controlled, for example, the usage ratio (such as volume ratio) of the anti-solvent to the good solvent may be 1-5:1. In a more preferred embodiment of the present disclosure, the usage ratio (such as volume ratio) of the anti-solvent to the good solvent may be 2:1.

The crystalline form II of the present disclosure can be prepared by the following preparation method. A crude product of 2-hydroxy-5-[2-(4-(trifluoromethylphenyl) ethylamino)]benzoic acid was added to a good solvent, stirred and heated until it is completely dissolved, and filtered while it was hot. The filtrate was subjected to rapid cooling crystallization or freeze-drying crystallization, thus obtaining the crystalline form II.

In an embodiment of the present disclosure, the good solvent for dissolving the crude product of 2-hydroxy-5-[2-(4-(trifluoromethylphenyl)ethylamino)]benzoic acid may be a single solvent or a mixture of two or more solvents in any ratio, and for example, may be at least one selected from the group consisting of methanol, ethanol and tetrahydrofuran. In a preferred embodiment of the present disclosure, the good solvent for dissolving the crude product of 2-hydroxy-5-[2-(4-(trifluoromethylphenyl)ethylamino)]benzoic acid may be at least one selected from the group consisting of methanol and ethanol.

In an embodiment of the present disclosure, the amount of the good solvent for dissolving the crude product of 2-hydroxy-5-[2-(4-(trifluoromethylphenyl)ethylamino)]benzoic acid can be controlled, for example, the usage ratio of the crude product to the good solvent may be 1 g: 5~300 mL.

In an embodiment of the present disclosure, the good solvent for dissolving the crude product of 2-hydroxy-5-[2-(4-(trifluoromethylphenyl)ethylamino)]benzoic acid can be treated by heating, for example, heating to 50~80°C.

As for the crystalline form II of the present disclosure, the target product can be obtained by various crystallization methods, such as rapid cooling crystallization, freeze-drying crystallization, etc.

In an embodiment of the present disclosure, the crystalline form II can be prepared by a rapid cooling crystallization method. Specifically, the target temperature of the rapid cooling crystallization may be 0°C or lower. In addition, the cooling rate of the rapid cooling crystallization may be 5°C/min or greater, that is, the temperature of the dissolved and clear filtrate of the crude product was decreased to the target temperature at a rate of decreasing 5°C per minute or faster.

In another embodiment of the present disclosure, the crystalline form II can be prepared by a freeze-drying crystallization method.

Further, the method for preparing the above crystalline forms I and II may optionally include post-treatment steps, such as filtration, drying, etc.

### Pharmaceutical composition

The crystalline forms I and II of 2-hydroxy-5-[2-(4-(trifluoromethylphenyl)ethylamino)]benzoic acid of the present disclosure can be administered in a pharmaceutical composition. The term "pharmaceutical composition" refers to a composition that can be used as a medicine, which comprises an active pharmaceutical ingredient (API) (for example, the crystalline forms I and II of the present disclosure) and optionally one or more pharmaceutically acceptable carriers. The term "pharmaceutically acceptable carrier" refers to a pharmaceutical excipient that is compatible with the active pharmaceutical ingredient and is harmless to a subject, including (but not limited to) diluents (or fillers), binders, disintegrants, lubricants, wetting agents, thickeners, glidants, flavoring agents, smelling agents, preservatives, antioxidants, pH regulators, solvents, cosolvents, surfactants, etc.

In one embodiment of the present disclosure, the pharmaceutical composition can comprise the crystalline form I of the present disclosure.

In another embodiment of the present disclosure, the pharmaceutical composition can comprise the crystalline form II of the present disclosure.

In another embodiment of the present disclosure, the pharmaceutical composition can comprise both the crystalline form I and the crystalline form II of the present disclosure, and the two crystalline forms can be mixed in any ratio.

In a preferred embodiment of the present disclosure, the above pharmaceutical composition further comprises at least one pharmaceutically acceptable carrier.

### Medical uses

Either the above crystalline form I or crystalline form II of 2-hydroxy-5-[2-(4-(trifluoromethylphenyl)ethylamino)]benzoic acid, or the above pharmaceutical composition containing the crystalline form I and/or crystalline form II, can be used to combat degenerative diseases of the central nervous system. Therefore, the present disclosure also provides the use of the above crystalline form I and/or crystalline form II in the preparation of a medicament for the prevention and/or treatment of degenerative diseases of the central nervous system.

The term "degenerative diseases of the central nervous system" refers to the general term for a group of diseases caused by chronic progressive degeneration of central nervous system. These diseases include (but are not limited to) Alzheimer's disease (AD), Parkinson's disease (PD), Huntington's disease (HD), and amyotrophic lateral sclerosis (ALS), etc.

In an embodiment of the present disclosure, the degenerative disease of the central nervous system may be at least one selected from the group consisting of Alzheimer's disease, Parkinson's disease, Huntington's disease and amyotrophic lateral sclerosis.

In a preferred embodiment of the present disclosure, the degenerative disease of the central nervous system may be Alzheimer's disease.

Hereinafter, the technical solutions of the present disclosure will be illustrated in conjunction with specific examples. It can be understood by those of ordinary skill in the art that the following examples are merely for further describing the present disclosure in detail, and do not limit the scope of the present disclosure. Unless otherwise specified, medicines, reagents, materials, instruments, etc. used in the following examples can be obtained by conventional commercial means.

**Example 1:** preparation and physicochemical identification of crystalline form I of the present disclosure.

### Step 1: preparation of dissolved and clear filtrate of crude product

A crude product (100 g) of 2-hydroxy-5-[2-(4-(trifluoromethylphenyl)ethylamino)] benzoic acid was weighed, and placed in a 1-L flask. Ethanol (500 mL) was added at room temperature, and the system was heated to 50°C under stirring. The system was subjected to suction filtration after being dissolved and cleared, and impurities were removed to obtain dissolved and clear filtrate of the crude product.

### Step 2: slow cooling crystallization:

The above dissolved and clear filtrate was cooled to -5°C at a cooling rate of 3°C/min, which was accompanied by the precipitation of white precipitates. The filtrate was kept at -5°C for 1 h until no precipitate was generated, and was subjected to suction filtration and drying, thereby obtaining the target product (the yield is 95.6%).

The results are shown in FIG. 1 by XRPD test and it is determined to be crystalline form I.

Crystalline form stability test: a certain amount of samples were left under 40°C and 60% RH conditions for three months, and their powder diffraction results were tested, as shown in FIG. 1. As can be seen from FIG. 1, the diffraction characteristic peak of the sample after long-term placement is consistent with that at initial time, indicating that the crystalline form is stable and unchanged, and the crystalline form I has long-term stability.

According to the DSC test, the melting occurred in the range of 249~252°C, as shown in FIG. 2.

A sample was taken and observed by SEM. The photograph is shown in FIG. 3, showing irregular large granular blocks.

The particle size and distribution of particles were tested by a particle size analyzer, and the average particle size is about 50 µm, as shown in FIG. 4.

**Example 2:** preparation and physicochemical identification of crystalline form I of the present disclosure.

### Step 1: preparation of dissolved and clear filtrate of crude product

A crude product (80 g) of 2-hydroxy-5-[2-(4-(trifluoromethylphenyl)ethylamino)] benzoic acid was weighed, and placed in a 1-L flask. Isopropanol (560 mL) was added at room temperature, and the system was heated to 80°C under stirring. The system was subjected to suction filtration after being dissolved and cleared, and impurities were removed to obtain dissolved and clear filtrate of the crude product.

### Step 2: evaporation of the solvent at a constant temperature for crystallization

The temperature of the above dissolved and clear filtrate was decreased to the ambient temperature of 30°C, and the solvent was naturally evaporated at this constant temperature, which was accompanied by the precipitation of white precipitates, until the solvent evaporated to the extent that no more precipitates were generated. The filtrate was subjected to suction filtration and drying, thereby obtaining the target product (the yield is 93.8%).

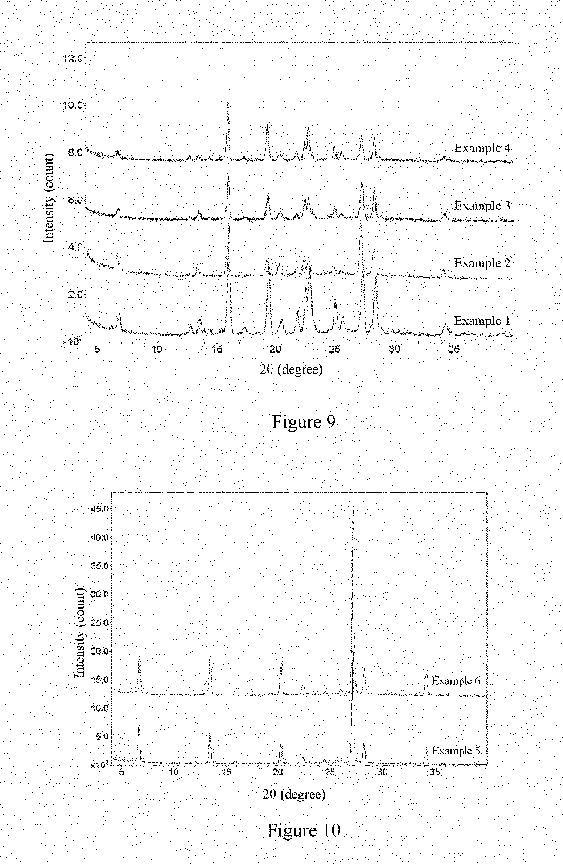

### It was determined to be crystalline form I by XRPD test, as shown in FIG. 9.

**Example 3:** preparation and physicochemical identification of crystalline form I of the present disclosure.

### Step 1: preparation of dissolved and clear filtrate of crude product

A crude product (100 g) of 2-hydroxy-5-[2-(4-(trifluoromethylphenyl)ethylamino)] benzoic acid was weighed, and placed in a 1-L flask. Isopropanol (800 mL) was added at room temperature, and the system was heated to 60°C under stirring. The system was subjected to suction filtration after being dissolved and cleared, and impurities were removed to obtain dissolved and clear filtrate of the crude product.

### Step 2: evaporation of the solvent at high temperature for crystallization

The above dissolved and clear filtrate was heated to 90°C, and the solvent was evaporated under a certain vacuum degree (-0.12 MPa), which was accompanied by the precipitation of white precipitates, until the solvent evaporated to the extent that no more precipitates were generated. The filtrate was subjected to suction filtration and drying, thereby obtaining the target product (the yield is 98.1%).

### It was determined to be crystalline form I by XRPD test, as shown in FIG. 9.

**Example 4:** preparation and physicochemical identification of crystalline form I of the present disclosure.

### Step 1: preparation of dissolved and clear filtrate of crude product

A crude product (50 g) of 2-hydroxy-5-[2-(4-(trifluoromethylphenyl)ethylamino)]benzoic acid was weighed, and placed in a 1-L flask. Ethanol (300 mL) was added at room temperature, and the system was heated to 60°C under stirring. The system was subjected to suction filtration after being dissolved and cleared, and impurities were removed to obtain dissolved and clear filtrate of the crude product.

### Step 2: addition of an anti-solvent for crystallization:

Water (600 mL) was slowly added to the above dissolved and clear filtrate, which was accompanied by the precipitation of white precipitates. The filtrate was subjected to suction filtration and drying, thereby obtaining the target product (the yield is 94.2%).

### It was determined to be crystalline form I by XRPD test, as shown in FIG. 9.

**Example 5:** preparation and physicochemical identification of crystalline form II of the present disclosure.

### Step 1: preparation of dissolved and clear filtrate of crude product

A crude product (80 g) of 2-hydroxy-5-[2-(4-(trifluoromethylphenyl)ethylamino)] benzoic acid was weighed, and placed in a 1-L flask. Methanol (400 mL) was added at room temperature, and the system was heated to 60°C under stirring. The system was subjected to suction filtration after being dissolved and cleared, and impurities were removed to obtain dissolved and clear filtrate of the crude product.

### Step 2: rapid cooling crystallization:

The above dissolved and clear filtrate was cooled to 0°C at a cooling rate of 8°C/min, which was accompanied by the precipitation of white precipitates. The filtrate was kept at 0°C until no precipitate was generated, and was subjected to suction filtration and drying, thereby obtaining the target product (the yield is 92.7%).

The results are shown in FIG. 5 by XRPD test and it is determined to be crystalline form II.

Crystalline form stability test: a certain amount of samples were left under 40°C and 60% RH conditions for three months, and their powder diffraction results were tested, as shown in FIG. 5. As can be seen from FIG. 5, the diffraction characteristic peak of the sample after long-term placement is consistent with that at initial time, indicating that the crystalline form is relatively stable, and the crystalline form II has certain long-term stability.

According to the DSC test, the melting occurred in the range of 247~250°C, as shown in FIG. 6.

A sample was taken and observed by SEM. The photograph is shown in FIG. 7, showing regular rod-shaped particles.

The particle size and distribution of particles were tested by a particle size analyzer, and the average particle size is about 18 µm, as shown in FIG. 8.

**Example 6:** preparation and physicochemical identification of crystalline form II of the present disclosure.

### Step 1: preparation of dissolved and clear filtrate of crude product

A crude product (100 g) of 2-hydroxy-5-[2-(4-(trifluoromethylphenyl)ethylamino)] benzoic acid was weighed, and placed in a 1-L flask. Ethanol (600 mL) was added at room temperature, and the system was heated to 50°C under stirring. The system was subjected to suction filtration after being dissolved and cleared, and impurities were removed to obtain dissolved and clear filtrate of the crude product.

### Step 2: freeze-drying crystallization:

The above dissolved and clear filtrate was frozen to a solid at -40°C, put into a freeze dryer, and taken out after vacuum drying to obtain the target product (the yield is 99.7%).

It was determined to be crystalline form II by XRPD test, as shown in FIG. 10.

In addition, the apparent solubility of crude raw materials and the crystalline form I prepared in Example 1 and the crystalline form II prepared in Example 5 were tested by HPLC, and the results show that the crystalline forms I and II of the present disclosure show higher apparent solubility, as shown in Table 2.

**Table 2. Comparison results of apparent solubility between crystalline forms I and II of the present disclosure and raw material**

| **Sample** | **Chromatographic peak area** | **Solubility (mg/mL)** |
|---|---|---|
| Raw material | 249333 | 1.77 |
| Crystalline form I | 263684 | 1.88 |
| Crystalline form II | 311308 | 2.60 |

## Claims

1. Crystalline form I of 2-hydroxy-5-[2-(4-(trifluoromethylphenyl)ethylamino)]benzoic acid, having the following crystallographic parameters, crystal system: monoclinic crystal system, space group: Pc, cell parameters: a=3.83880(10)Å, b=8.82524(12)Å, c=11.62736(9)Å, α=90°, β=98.7472°, γ=90°, and number of molecules per unit cell: Z=2.

2. The crystalline form I according to claim 1, **characterized in that** its X-ray powder diffraction spectrum shows characteristic peaks at 2θ values of 16.1±0.2°, 19.4±0.2°, 22.9±0.2°, 27.3±0.2° and 28.4±0.2°, preferably its X-ray powder diffraction spectrum shows characteristic peaks at 2θ values of at least one of 6.9±0.2°, 13.6±0.2°, 20.5±0.2°, 21.9±0.2°, 22.6±0.2°, 25.1±0.2°, 25.7±0.2° and 34.3±0.2°, more preferably its X-ray powder diffraction spectrum shows characteristic peaks at 2θ values of at least one of 12.9±0.2°, 17.4±0.2°, 29.0±0.2°, 29.7±0.2°, 31.4±0.2°, 34.8±0.2°, 36.0±0.2°, 36.5±0.2° and 39.1±0.2°, and most preferably its X-ray powder diffraction spectrum is shown in FIG. 1.

3. The crystalline form I according to claim 1 or 2, **characterized in that** its differential scanning calorimetry analysis graph shows an endothermic peak at 249-252°C, and preferably its differential scanning calorimetry analysis graph is shown in FIG. 2.

4. Crystalline form II of 2-hydroxy-5-[2-(4-(trifluoromethylphenyl)ethylamino)]benzoic acid, having the following crystallographic parameters, crystal system: triclinic crystal system, space group: P1, cell parameters: a=4.47850(8)Å, b=6.31509(15)Å, c=13.34065(22)Å, α=89.9804(7)°, β=79.7108(12)°, γ=80.6128(8)°, and number of molecules per unit cell: Z=2.

5. The crystalline form II according to claim 4, **characterized in that** its X-ray powder diffraction spectrum shows characteristic peaks at 2θ values of 6.8±0.2°, 13.5±0.2°, 27.2±0.2° and 28.3±0.2°, preferably its X-ray powder diffraction spectrum shows characteristic peaks at 2θ values of at least one of 16.0±0.2°, 20.4±0.2°, 22.4±0.2° and 34.2±0.2°, more preferably its X-ray powder diffraction spectrum shows characteristic peaks at 2θ values of at least one of 19.3±0.2°, 22.7±0.2°, 23.1±0.2°, 24.5±0.2°, 25.0±0.2° and 26.0±0.2°, and most preferably its X-ray powder diffraction spectrum is shown in FIG. 5.

6. The crystalline form II according to claim 4 or 5, **characterized in that** its differential scanning calorimetry analysis graph shows an endothermic peak at 247~250°C, and preferably its differential scanning calorimetry analysis graph is shown in FIG. 6.

7. A method for preparing the crystalline form I according to any one of claims 1 to 3, comprising:
adding a crude product of 2-hydroxy-5-[2-(4-(trifluoromethylphenyl)ethylamino)]benzoic acid to a good solvent, stirring and heating until it is completely dissolved, filtering while it is hot, and subjecting the filtrate to slow cooling crystallization, evaporation of the solvent at a constant temperature for crystallization, evaporation of the solvent at an increased temperature for crystallization or addition of an anti-solvent for crystallization to obtain the crystalline form I.

8. A method for preparing the crystalline form II according to any one of claims 4 to 6, comprising:
adding a crude product of 2-hydroxy-5-[2-(4-(trifluoromethylphenyl)ethylamino)]benzoic acid to a good solvent, stirring and heating until it is completely dissolved, filtering while it is hot, and subjecting the filtrate to rapid cooling crystallization or freeze-drying crystallization to obtain the crystalline form II.

9. A pharmaceutical composition comprising the crystalline form I according to any one of claims 1 to 3 and/or the crystalline form II according to any one of claims 4 to 6, and at least one pharmaceutically acceptable carrier.

10. Use of the crystalline form I according to any one of claims 1 to 3 and/or the crystalline form II according to any one of claims 4 to 6 in the preparation of a medicament for the prevention and/or treatment of degenerative diseases of the central nervous system.
